# EUROPEAN PATENT SPECIFICATION

(11) **EP 4 175 549 B1**
(45) Date of publication and mention of the grant of the patent: **24.12.2025**
(21) Application number: 21756155.4
(22) Date of filing: 29.07.2021
(51) Int. Cl.: A61B 5/388, A61N 1/05, A61N 1/36, A61B 5/00, A61B 34/20, A61N 1/372

(54) **ELECTRICAL STIMULATION SYSTEMS BASED ON STIMULATION-EVOKED RESPONSES**
ELEKTRISCHE STIMULATIONSSYSTEME AUF BASIS VON STIMULATIONSERMÖGLICHTEN REAKTIONEN
SYSTÈMES DE STIMULATION ÉLECTRIQUE BASÉS SUR DES RÉPONSES ÉVOQUÉES PAR STIMULATION

(30) Priority: 10.08.2020 US 202063063706 P
(43) Date of publication of application: 10.05.2023
(73) Proprietor: Boston Scientific Neuromodulation Corporation, Valencia, CA 91355 (US)
(72) Inventor: MOFFITT, Michael, Solon, OH 44139 (US)
(74) Representative: Vossius & Partner Patentanwälte Rechtsanwälte mbB
(86) International application number: PCT/US2021/043705
(87) International publication number: WO 2022/035608

(56) References cited:
- WO-A2-2018/008034
- US-A1- 2014 163 639
- US-A1- 2014 277 282
- US-A1- 2016 361 542
- US-A1- 2018 140 843
- US-A1- 2019 143 120
- US-A1- 2020 138 324

## Description

### FIELD OF THE INVENTION

This application relates to deep brain stimulation (DBS), and more particularly, to methods and systems for using sensed evoked resonant neural responses (ERNA) for facilitating aspects of DBS.

### INTRODUCTION

Implantable neurostimulator devices are devices that generate and deliver electrical stimuli to body nerves and tissues for the therapy of various biological disorders, such as pacemakers to treat cardiac arrhythmia, defibrillators to treat cardiac fibrillation, cochlear stimulators to treat deafness, retinal stimulators to treat blindness, muscle stimulators to produce coordinated limb movement, spinal cord stimulators to treat chronic pain, cortical and deep brain stimulators to treat motor and psychological disorders, and other neural stimulators to treat urinary incontinence, sleep apnea, shoulder subluxation, etc. The description that follows will generally focus on the use of the invention within a Deep Brain Stimulation (DBS). DBS has been applied therapeutically for the treatment of neurological disorders, including Parkinson's Disease, essential tremor, dystonia, and epilepsy, to name but a few. Further details discussing the treatment of diseases using DBS are disclosed in U.S. Pat. Nos. 6,845,267, 6,845,267, and 6,950,707. However, the present invention may find applicability with any implantable neurostimulator device system.

Each of these implantable neurostimulation systems typically includes one or more electrode carrying stimulation leads, which are implanted at the desired stimulation site, and a neurostimulator implanted remotely from the stimulation site, but coupled either directly to the neurostimulation lead(s) or indirectly to the neurostimulation lead(s) via a lead extension. The neurostimulation system may further comprise a handheld external control device to remotely instruct the neurostimulator to generate electrical stimulation pulses in accordance with selected stimulation parameters. Typically, the stimulation parameters programmed into the neurostimulator can be adjusted by manipulating controls on the external control device to modify the electrical stimulation provided by the neurostimulator system to the patient.

Thus, in accordance with the stimulation parameters programmed by the external control device, electrical pulses can be delivered from the neurostimulator to the stimulation electrode(s) to stimulate or activate a volume of tissue in accordance with a set of stimulation parameters and provide the desired efficacious therapy to the patient. The best stimulus parameter set will typically be one that delivers stimulation energy to the volume of tissue that must be stimulated in order to provide the therapeutic benefit (e.g., treatment of movement disorders), while minimizing the volume of non-target tissue that is stimulated. A typical stimulation parameter set may include the electrodes that are acting as anodes or cathodes, as well as the amplitude, duration, and rate of the stimulation pulses.

Non-optimal electrode placement and stimulation parameter selections may result in excessive energy consumption due to stimulation that is set at too high an amplitude, too wide a pulse duration, or too fast a frequency; inadequate or marginalized treatment due to stimulation that is set at too low an amplitude, too narrow a pulse duration, or too slow a frequency; or stimulation of neighboring cell populations that may result in undesirable side effects. For example, bilateral DBS of the subthalamic nucleus has been proven to provide effective therapy for improving the major motor signs of advanced Parkinson's disease, and although the bilateral stimulation of the subthalamic nucleus is considered safe, an emerging concern is the potential negative consequences that it may have on cognitive functioning and overall quality of life (see A. M. M. Frankemolle, et al., Reversing Cognitive-Motor Impairments in Parkinson's Disease Patients Using a Computational Modelling Approach to Deep Brain Stimulation Programming, Brain 2010; pp. 1-16). In large part, this phenomenon is due to the small size of the subthalamic nucleus. Even with the electrodes are located predominately within the sensorimotor territory, the electrical field generated by DBS is non-discriminately applied to all neural elements surrounding the electrodes, thereby resulting in the spread of current to neural elements affecting cognition. As a result, diminished cognitive function during stimulation of the subthalamic nucleus may occur do to non-selective activation of non-motor pathways within or around the subthalamic nucleus.

The large number of electrodes available, combined with the ability to generate a variety of complex stimulation pulses, presents a huge selection of stimulation parameter sets to the clinician or patient. In the context of DBS, neurostimulation leads with a complex arrangement of electrodes that not only are distributed axially along the leads, but are also distributed circumferentially around the neurostimulation leads as segmented electrodes, can be used.

To facilitate such selection, the clinician generally programs the external control device, and if applicable the neurostimulator, through a computerized programming system. This programming system can be a self-contained hardware/software system, or can be defined predominantly by software running on a standard personal computer (PC). The PC or custom hardware may actively control the characteristics of the electrical stimulation generated by the neurostimulator to allow the optimum stimulation parameters to be determined based on patient feedback and to subsequently program the external control device with the optimum stimulation parameters.

When electrical leads are implanted within the patient, the computerized programming system may be used to instruct the neurostimulator to apply electrical stimulation to test placement of the leads and/or electrodes, thereby assuring that the leads and/or electrodes are implanted in effective locations within the patient. Once the leads are correctly positioned, a fitting procedure, which may be referred to as a navigation session, may be performed using the computerized programming system to program the external control device, and if applicable the neurostimulator, with a set of stimulation parameters that best addresses the neurological disorder(s).

In the context of DBS, the brain is dynamic (e.g., due to disease progression, motor re-learning, or other changes), and a program (i.e., a set of stimulation parameters) that is useful for a period of time may not maintain its effectiveness and/or the expectations of the patient may increase. Further, physicians typically treat the patient with stimulation and medication, and proper amounts of each are required for optimal therapy. In particular, a patient's stimulation needs may be impacted by their medication state.

Thus, there is a need for methods and systems that assist a clinician in obtaining an optimum lead placement during implantation process and to determine optimum stimulation parameters for treating the patient. There is also a need for closed loop feedback that can be used to adjust stimulation parameters as the patient's stimulation needs change with time or based on their medication state. US2019/143120 discloses systems and methods for monitoring neural activity.

### SUMMARY OF THE DESCRIPTION

Disclosed herein is a method of implanting a stimulation lead in the brain of a patient, wherein the stimulation lead comprises a plurality of electrodes, the method comprising: positioning the lead at a first position in the patient's brain, determining a plurality of stimulation locations at which to apply stimulation, wherein at least one of the stimulation locations is not co-located with an electrode, using the electrodes to sequentially apply stimulation at each of the determined stimulation locations, wherein applying stimulation at the at least one location not co-located with an electrode comprises fractionalizing current to two or more electrodes to provide the stimulation at the determined stimulation location, detecting an evoked response evoked at a neural target for each of the stimulation locations, and determining one or more of (i) whether to move the lead to a new position or (ii) to adjust stimulation parameters based on the evoked responses. According to some embodiments, the detected evoke response occurs following an evoked compound action potential (ECAP). According to some embodiments, at least two electrodes of the same polarity are used during at least one stimulation. According to some embodiments, the evoked response is evoked resonant neural activity (ERNA). According to some embodiments, the method further comprises displaying an indication of the ERNAs for each of the stimulation locations on a user interface (UI). According to some embodiments, the method further comprises displaying an indication of the ERNA interpolated between the stimulation locations on the user interface. According to some embodiments, the indications of the ERNAs for each of the stimulation locations comprises an indication of the ERNA amplitudes. According to some embodiments, the UI comprises a representation of the stimulation lead and wherein the indications of the ERNA amplitudes are displayed upon the representation of the stimulation lead at positions corresponding to the corresponding stimulation locations. According to some embodiments the method further comprises determining a prediction of efficacy for stimulation using the lead at the first position. According to some embodiments, the method further comprises determining a relative position of the first position with respect to the neural target. According to some embodiments, determining the relative position of the first position with respect to the neural target comprises estimating a distance between the first position and the neural target. According to some embodiments, determining the relative position of the first position with respect to the neural target comprises determining a direction from the first position to the neural target. According to some embodiments, the method further comprises determining an orientation of the lead with respect to the neural target. According to some embodiments, using the electrodes to sequentially apply stimulation at a plurality of stimulation locations comprises applying monopolar stimulation. According to some embodiments, applying monopolar stimulation comprises applying a first set of monopolar stimulation pulses of a first polarity and a second set of monopolar stimulation pulses of a second polarity. According to some embodiments, the method further comprises comparing ERNA responses evoked by the stimulation pulses of the first polarity and ERNA responses evoked by the stimulation pulses of the second polarity. According to some embodiments, the method further comprises determining an orientation of the lead with respect to the neural target based on the comparison.

Also disclosed herein is a method of providing deep brain stimulation (DBS) to a patient using a stimulation lead in the brain of the patient, wherein the stimulation lead comprises a plurality of electrodes, the method comprising: applying electrical stimulation to the patient's brain using one or more of a plurality of electrodes configured on an electrode lead implanted in the patient's brain, wherein the electrical stimulation comprises a first phase comprising a plurality of monophasic pulses of a first amplitude and a first polarity followed by a quiescent period, and during the quiescent period, detecting evoked neural responses evoked within a region of neural tissue within the patient's brain by the stimulation. According to some embodiments, the evoked neural responses are resonant neural activity (ERNA). According to some embodiments, the electrical stimulation further comprises a second phase comprising at least one pulse of a second amplitude and a second polarity opposite of the first polarity. According to some embodiments, the second amplitude is less than the first amplitude. According to some embodiments, the first phase and the second phase are charge balanced. According to some embodiments, the first phase precedes the second phase and wherein the quiescent period is between the first phase and the second phase. According to some embodiments, the second phase precedes the first phase. According to some embodiments, the method further comprises determining one or more parameters of the ERNAs. According to some embodiments, the one or more parameters comprise frequency, rate of decay, number of pulses or amplitude. According to some embodiments, the method further comprises adjusting the electrical stimulation based on the one or more parameters of the ERNAs.

Also disclosed herein is an apparatus for facilitating the implantation of a stimulation lead in the brain of a patient, wherein the stimulation lead comprises a plurality of electrodes, the apparatus comprising control circuitry configured to: receive an indication that the lead is at a first position in the patient's brain, determine a plurality of stimulation locations at which to apply stimulation, wherein at least one of the stimulation locations is not co-located with an electrode, apply stimulation at each of the determined stimulation locations, wherein applying stimulation at the at least one location not co-located with an electrode comprises fractionalizing current to two or more electrodes to provide the stimulation at the determined stimulation location, detect an evoked response evoked at a neural target for each of the stimulation locations, and determine one or more of (i) whether to move the lead to a new position or (ii) to adjust stimulation parameters based on the evoked responses. According to some embodiments, the detected evoke response occurs following an evoked compound action potential (ECAP). According to some embodiments, at least two electrodes of the same polarity are used during at least one stimulation. According to some embodiments, the evoked response is evoked resonant neural activity (ERNA). According to some embodiments, the control circuitry is further configured to display an indication of the ERNAs for each of the stimulation locations on a user interface (UI). According to some embodiments, the control circuitry is further configured to display an indication of the ERNA interpolated between the stimulation locations on the user interface. According to some embodiments, the indications of the ERNAs for each of the stimulation locations comprises an indication of the ERNA amplitudes. According to some embodiments, the UI comprises a representation of the stimulation lead and wherein the indications of the ERNA amplitudes are displayed upon the representation of the stimulation lead at positions corresponding to the corresponding stimulation locations. According to some embodiments, the control circuitry is further configured to determine a prediction of efficacy for stimulation using the lead at the first position. According to some embodiments, the control circuitry is further configured to determine a relative position of the first position with respect to the neural target. According to some embodiments, determining the relative position of the first position with respect to the neural target comprises estimating a distance between the first position and the neural target. According to some embodiments, determining the relative position of the first position with respect to the neural target comprises determining a direction from the first position to the neural target. According to some embodiments, the control circuitry is further configured to determine an orientation of the lead with respect to the neural target. According to some embodiments, using the electrodes to sequentially apply stimulation at a plurality of stimulation locations comprises applying monopolar stimulation. According to some embodiments, applying monopolar stimulation comprises applying a first set of monopolar stimulation pulses of a first polarity and a second set of monopolar stimulation pulses of a second polarity. According to some embodiments, the control circuitry is further configured to compare ERNA responses evoked by the stimulation pulses of the first polarity and ERNA responses evoked by the stimulation pulses of the second polarity. According to some embodiments, the control circuitry is further configured to determine an orientation of the lead with respect to the neural target based on the comparison.

Also disclosed herein is an apparatus for providing deep brain stimulation (DBS) to a patient using a stimulation lead in the brain of the patient, wherein the stimulation lead comprises a plurality of electrodes, the apparatus comprising control circuitry configured to: apply electrical stimulation to the patient's brain using one or more of a plurality of electrodes configured on an electrode lead implanted in the patient's brain, wherein the electrical stimulation comprises a first phase comprising a plurality of monophasic pulses of a first amplitude and a first polarity followed by a quiescent period, and during the quiescent period, detect evoked neural responses evoked within a region of neural tissue within the patient's brain by the stimulation. According to some embodiments, the is evoked neural responses are resonant neural activity (ERNA). According to some embodiments, the electrical stimulation further comprises a second phase comprising at least one pulse of a second amplitude and a second polarity opposite of the first polarity. According to some embodiments, the second amplitude is less than the first amplitude. According to some embodiments, the first phase and the second phase are charge balanced. According to some embodiments, the first phase precedes the second phase and wherein the quiescent period is between the first phase and the second phase. According to some embodiments, the second phase precedes the first phase. According to some embodiments, the control circuitry is further configured to determine one or more parameters of the ERNAs. According to some embodiments, the one or more parameters comprise frequency, rate of decay, number of pulses or amplitude. According to some embodiments, the control circuitry if further configured to adjust the electrical stimulation based on the one or more parameters of the ERNAs.

Also disclosed herein is a non-transitory computer readable medium for facilitating the implantation of a stimulation lead in the brain of a patient, wherein the stimulation lead comprises a plurality of electrodes, the non-transitory computer readable medium comprising: instructions that are executable by control circuitry of an external device to cause the control circuitry to: receive an indication that the lead is at a first position in the patient's brain, determine a plurality of stimulation locations at which to apply stimulation, wherein at least one of the stimulation locations is not co-located with an electrode, apply stimulation at each of the determined stimulation locations, wherein applying stimulation at the at least one location not co-located with an electrode comprises fractionalizing current to two or more electrodes to provide the stimulation at the determined stimulation location, detect an evoked response evoked at a neural target for each of the stimulation locations, and determine one or more of (i) whether to move the lead to a new position or (ii) to adjust stimulation parameters based on the evoked responses. According to some embodiments, the detected evoke response occurs following an evoked compound action potential (ECAP). According to some embodiments, at least two electrodes of the same polarity are used during at least one stimulation. According to some embodiments, the evoked response is evoked resonant neural activity (ERNA). According to some embodiments, the control circuitry is further configured to display an indication of the ERNAs for each of the stimulation locations on a user interface (UI). According to some embodiments, the control circuitry is further configured to display an indication of the ERNA interpolated between the stimulation locations on the user interface. According to some embodiments, the indications of the ERNAs for each of the stimulation locations comprises an indication of the ERNA amplitudes. According to some embodiments, the UI comprises a representation of the stimulation lead and wherein the indications of the ERNA amplitudes are displayed upon the representation of the stimulation lead at positions corresponding to the corresponding stimulation locations. According to some embodiments, the control circuitry is further configured to determine a prediction of efficacy for stimulation using the lead at the first position. According to some embodiments, the control circuitry is further configured to determine a relative position of the first position with respect to the neural target. According to some embodiments, determining the relative position of the first position with respect to the neural target comprises estimating a distance between the first position and the neural target. According to some embodiments, determining the relative position of the first position with respect to the neural target comprises determining a direction from the first position to the neural target. According to some embodiments, the control circuitry is further configured to determine an orientation of the lead with respect to the neural target. According to some embodiments, using the electrodes to sequentially apply stimulation at a plurality of stimulation locations comprises applying monopolar stimulation. According to some embodiments, applying monopolar stimulation comprises applying a first set of monopolar stimulation pulses of a first polarity and a second set of monopolar stimulation pulses of a second polarity. According to some embodiments, the control circuitry is further configured to compare ERNA responses evoked by the stimulation pulses of the first polarity and ERNA responses evoked by the stimulation pulses of the second polarity. According to some embodiments, the control circuitry is further configured to determine an orientation of the lead with respect to the neural target based on the comparison.

The invention may also reside in the form of a programed external device (via its control circuitry) for carrying out the above methods, a programmed IPG or ETS (via its control circuitry) for carrying out the above methods, a system including a programmed external device and IPG or ETS for carrying out the above methods, or as a computer readable media for carrying out the above methods stored in an external device or IPG or ETS. Examples of computer readable media include one or more non-transitory computer-readable storage mediums including, for example, magnetic disks (fixed, floppy, and removable) and tape, optical media such as CD-ROMs and digital versatile disks (DVDs), and semiconductor memory devices such as Electrically Programmable Read-Only Memory (EPROM), Electrically Erasable Programmable Read-Only Memory (EEPROM), and USB or thumb drive.

### BRIEF DESCRIPTION OF THE DRAWINGS

Figure 1A shows an Implantable Pulse Generator (IPG), in accordance with the prior art.
Figure 1B shows a percutaneous lead having split-ring electrodes, in accordance with the prior art.
Figures 2A and 2B show an example of stimulation pulses (waveforms) producible by the IPG or by an External Trial Stimulator (ETS), in accordance with the prior art.
Figure 3 shows an example of stimulation circuitry useable in the IPG or ETS, in accordance with the prior art.
Figure 4 shows an ETS environment useable to provide stimulation before implantation of an IPG, in accordance with the prior art.
Figure 5 shows various external devices capable of communicating with and programming stimulation in an IPG or ETS, in accordance with the prior art.
Figure 6 illustrates sensing circuitry useable in an IPG.
Figure 7 illustrates an embodiment of a user interface (UI) for programming stimulation.
Figure 8 illustrates evoked resonant neural activity (ERNA).
Figure 9 illustrates a system for using ERNA for surgical support during electrode lead implantation.
Figure 10 illustrates an example UI page showing ERNA responses to various electrode stimulation configurations.
Figure 11 illustrates an example workflow for using ERNA for surgical support during electrode lead implantation.
Figures 12 A and 12 B illustrate source location of neural elements.
Figure 13 illustrates selective activation order of regions of a neuron based on electrode position and stimulation polarity.
Figure 14 illustrates various waveforms for stimulation and for evoking and sensing ERNA responses.
Figure 15 illustrates a workflow for optimizing therapy and for correlating ERNA features with patient state.
Figure 16 illustrates a workflow for closed-loop control of stimulation using ERNA parameters.

### DETAILED DESCRIPTION

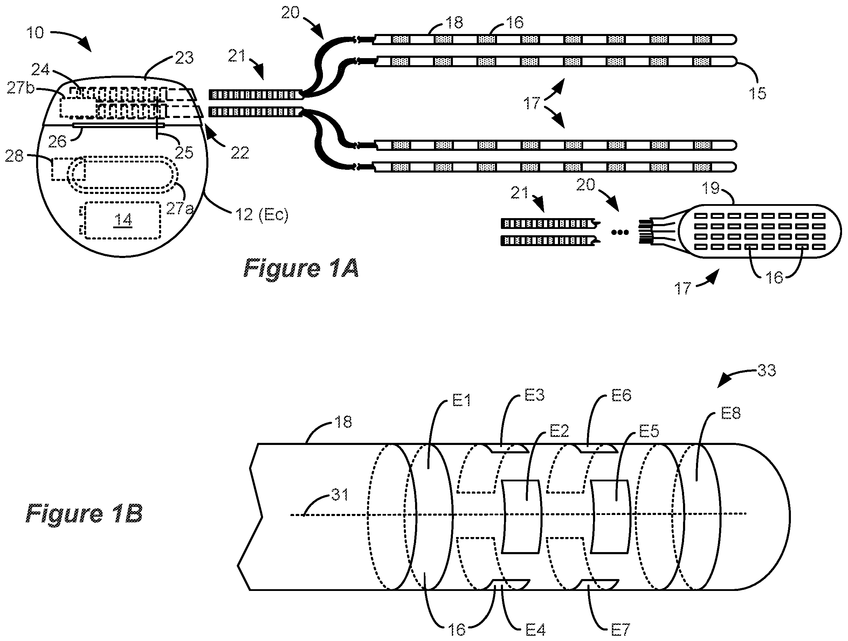

A DBS or SCS system typically includes an Implantable Pulse Generator (IPG) 10 shown in Figure 1A. The IPG 10 includes a biocompatible device case 12 that holds the circuitry and a battery 14 for providing power for the IPG to function. The IPG 10 is coupled to tissue-stimulating electrodes 16 via one or more electrode leads that form an electrode array 17. For example, one or more percutaneous leads 15 can be used having ring-shaped electrodes 16 carried on a flexible body 18. In another example, a paddle lead 19 provides electrodes 16 positioned on one of its generally flat surfaces.

In yet another example shown in Figure 1B, a lead 33 can include one or more split-ring electrodes. In this example, eight electrodes 16 (E1-E8) are shown. Electrode E8 at the distal end of the lead and electrode E1 at a proximal end of the lead comprise ring electrodes spanning 360 degrees around a central axis of the lead 33. Electrodes E2, E3, and E4 comprise split-ring electrodes, each of which are located at the same longitudinal position along the central axis 31, but with each spanning less than 360 degrees around the axis. For example, each of electrodes E2, E3, and E4 may span 90 degrees around the axis 31, with each being separated from the others by gaps of 30 degrees. Electrodes E5, E6, and E7 also comprise split-ring electrodes, but are located at a different longitudinal position along the central axis 31 than are split ring electrodes E1, E2, and E3. As shown, the split-ring electrodes E1-E3 and E5-E7 may be located at longitudinal positions along the axis 31 between ring electrodes E1 and E8. However, this is just one example of a lead 33 having split-ring electrodes. In other designs, all electrodes can be split-ring, or there could be different numbers of split-ring electrodes at each longitudinal position (i.e., more or less than three), or the ring and split-ring electrodes could occur at different or random longitudinal positions, etc.

Lead wires 20 within the leads are coupled to the electrodes 16 and to proximal contacts 21 insertable into lead connectors 22 fixed in a header 23 on the IPG 10, which header can comprise an epoxy for example. Once inserted, the proximal contacts 21 connect to header contacts 24 within the lead connectors 22, which are in turn coupled by feedthrough pins 25 through a case feedthrough 26 to stimulation circuitry 28 within the case 12, which stimulation circuitry 28 is described below.

In the IPG 10 illustrated in Figure 1A, there are thirty-two electrodes (E1-E32), split between four percutaneous leads 15, or contained on a single paddle lead 19, and thus the header 23 may include a 2x2 array of eight-electrode lead connectors 22. However, the type and number of leads, and the number of electrodes, in an IPG is application specific and therefore can vary. The conductive case 12 can also comprise an electrode (Ec).

In a SCS application, as is useful to alleviate chronic back pain for example, the electrode lead(s) are typically implanted in the spinal column proximate to the dura in a patient's spinal cord, preferably spanning left and right of the patient's spinal column. The proximal contacts 21 are tunneled through the patient's tissue to a distant location such as the buttocks where the IPG case 12 is implanted, at which point they are coupled to the lead connectors 22. In a DBS application, as is useful in the treatment of tremor in Parkinson's disease for example, the IPG 10 is typically implanted under the patient's clavicle (collarbone). Percutaneous leads 15 are tunneled through the neck and the scalp where the electrodes 16 are implanted through holes drilled in the skull and positioned for example in the subthalamic nucleus (STN) and the pedunculopontine nucleus (PPN) in each brain hemisphere. In other IPG examples designed for implantation directly at a site requiring stimulation, the IPG can be lead-less, having electrodes 16 instead appearing on the body of the IPG 10. The IPG lead(s) can be integrated with and permanently connected to the IPG 10 in other solutions.

IPG 10 can include an antenna 27a allowing it to communicate bi-directionally with a number of external devices discussed subsequently. Antenna 27a as shown comprises a conductive coil within the case 12, although the coil antenna 27a can also appear in the header 23. When antenna 27a is configured as a coil, communication with external devices preferably occurs using near-field magnetic induction. IPG 10 may also include a Radio-Frequency (RF) antenna 27b. In Figure 1A, RF antenna 27b is shown within the header 23, but it may also be within the case 12. RF antenna 27b may comprise a patch, slot, or wire, and may operate as a monopole or dipole. RF antenna 27b preferably communicates using far-field electromagnetic waves, and may operate in accordance with any number of known RF communication standards, such as Bluetooth, Bluetooth Low Energy (BLE), as described in U.S. Patent Publication 2019/0209851, Zigbee, WiFi, MICS, and the like.

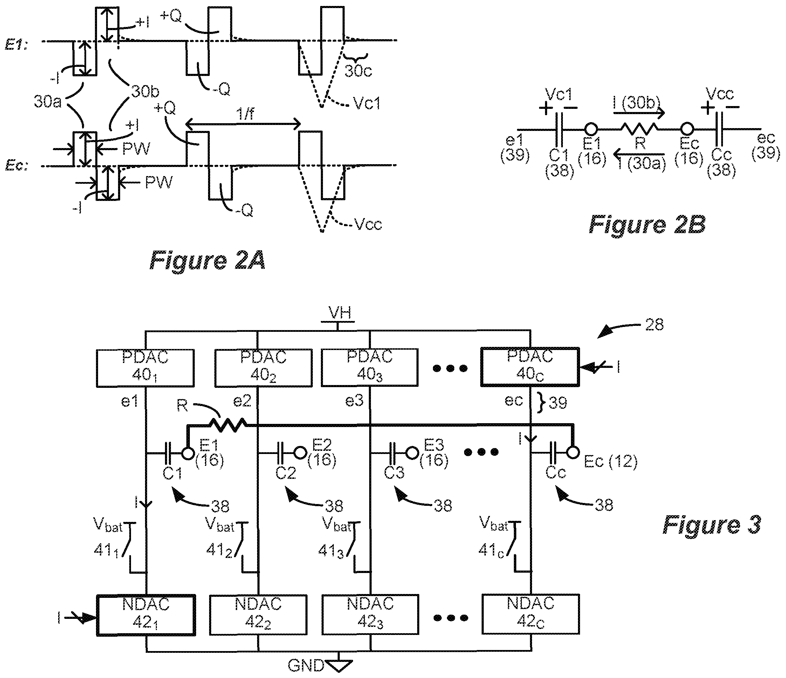

Stimulation in IPG 10 is typically provided by pulses each of which may include a number of phases such as 30a and 30b, as shown in the example of Figure 2A. In the example shown, such stimulation is monopolar, meaning that a current is provided between at least one selected lead-based electrode (e.g., E1) and the case electrode Ec 12. Stimulation parameters typically include amplitude (current I, although a voltage amplitude V can also be used); frequency (f); pulse width (PW) of the pulses or of its individual phases such as 30a and 30b; the electrodes 16 selected to provide the stimulation; and the polarity of such selected electrodes, i.e., whether they act as anodes that source current to the tissue or cathodes that sink current from the tissue. These and possibly other stimulation parameters taken together comprise a stimulation program that the stimulation circuitry 28 in the IPG 10 can execute to provide therapeutic stimulation to a patient.

In the example of Figure 2A, electrode E1 has been selected as a cathode (during its first phase 30a), and thus provides pulses which sink a negative current of amplitude -I from the tissue. The case electrode Ec has been selected as an anode (again during first phase 30a), and thus provides pulses which source a corresponding positive current of amplitude +I to the tissue. Note that at any time the current sunk from the tissue (e.g., -I at E1 during phase 30a) equals the current sourced to the tissue (e.g., +I at Ec during phase 30a) to ensure that the net current injected into the tissue is zero. The polarity of the currents at these electrodes can be changed: Ec can be selected as a cathode, and E1 can be selected as an anode, etc.

IPG 10 as mentioned includes stimulation circuitry 28 to form prescribed stimulation at a patient's tissue. Figure 3 shows an example of stimulation circuitry 28, which includes one or more current sources 40ᵢ and one or more current sinks 42ᵢ. The sources and sinks 40ᵢ and 42ᵢ can comprise Digital-to-Analog converters (DACs), and may be referred to as PDACs 40ᵢ and NDACs 42ᵢ in accordance with the Positive (sourced, anodic) and Negative (sunk, cathodic) currents they respectively issue. In the example shown, a NDAC/PDAC 40ᵢ/42ᵢ pair is dedicated (hardwired) to a particular electrode node ei 39. Each electrode node ei 39 is connected to an electrode Ei 16 via a DC-blocking capacitor Ci 38, for the reasons explained below. PDACs 40ᵢ and NDACs 42ᵢ can also comprise voltage sources.

Proper control of the PDACs 40ᵢ and NDACs 42ᵢ allows any of the electrodes 16 and the case electrode Ec 12 to act as anodes or cathodes to create a current through a patient's tissue, R, hopefully with good therapeutic effect. In the example shown, and consistent with the first pulse phase 30a of Figure 2A, electrode E1 has been selected as a cathode electrode to sink current from the tissue R and case electrode Ec has been selected as an anode electrode to source current to the tissue R. Thus PDAC 40c and NDAC 42₁ are activated and digitally programmed to produce the desired current, I, with the correct timing (e.g., in accordance with the prescribed frequency F and pulse width PW). Power for the stimulation circuitry 28 is provided by a compliance voltage VH, as described in further detail in U.S. Patent Application Publication 2013/0289665.

Other stimulation circuitries 28 can also be used in the IPG 10. In an example not shown, a switching matrix can intervene between the one or more PDACs 40ᵢ and the electrode nodes ei 39, and between the one or more NDACs 42ᵢ and the electrode nodes. Switching matrices allows one or more of the PDACs or one or more of the NDACs to be connected to one or more electrode nodes at a given time. Various examples of stimulation circuitries can be found in USPs 6,181,969, 8,606,362, 8,620,436, U.S. Patent Application Publications 2018/0071520 and 2019/0083796. The stimulation circuitries described herein provide multiple independent current control (MICC) (or multiple independent voltage control) to guide the estimate of current fractionalization among multiple electrodes and estimate a total amplitude that provide a desired strength. In other words, the total anodic current can be split among two or more electrodes and/or the total cathodic current can be split among two or more electrodes, allowing the stimulation location and resulting field shapes to be adjusted.

Much of the stimulation circuitry 28 of Figure 3, including the PDACs 40ᵢ and NDACs 42ᵢ, the switch matrices (if present), and the electrode nodes ei 39 can be integrated on one or more Application Specific Integrated Circuits (ASICs), as described in U.S. Patent Application Publications 2012/0095529, 2012/0092031, and 2012/0095519. As explained in these references, ASIC(s) may also contain other circuitry useful in the IPG 10, such as telemetry circuitry (for interfacing off chip with telemetry antennas 27a and/or 27b), circuitry for generating the compliance voltage VH, various measurement circuits, etc.

Also shown in Figure 3 are DC-blocking capacitors Ci 38 placed in series in the electrode current paths between each of the electrode nodes ei 39 and the electrodes Ei 16 (including the case electrode Ec 12). The DC-blocking capacitors 38 act as a safety measure to prevent DC current injection into the patient, as could occur for example if there is a circuit fault in the stimulation circuitry 28. The DC-blocking capacitors 38 are typically provided off-chip (off of the ASIC(s)), and instead may be provided in or on a circuit board in the IPG 10 used to integrate its various components, as explained in U.S. Patent Application Publication 2015/0157861.

Referring again to Figure 2A, the stimulation pulses as shown are biphasic, with each pulse comprising a first phase 30a followed thereafter by a second phase 30b of opposite polarity. Biphasic pulses are useful to actively recover any charge that might be stored on capacitive elements in the electrode current paths, such as on the DC-blocking capacitors 38. Charge recovery is shown with reference to both Figures 2A and 2B. During the first pulse phase 30a, charge will build up across the DC-blockings capacitors C1 and Cc associated with the electrodes E1 and Ec used to produce the current, giving rise to voltages Vc1 and Vcc which decrease in accordance with the amplitude of the current and the capacitance of the capacitors 38 (dV/dt = I/C). During the second pulse phase 30b, when the polarity of the current I is reversed at the selected electrodes E1 and Ec, the stored charge on capacitors C1 and Cc is actively recovered, and thus voltages Vc1 and Vcc increase and return to 0V at the end the second pulse phase 30b.

To recover all charge by the end of the second pulse phase 30b of each pulse (Vc1 = Vcc = 0V), the first and second phases 30a and 30b are charged balanced at each electrode, with the first pulse phase 30a providing a charge of -Q (-I * PW) and the second pulse phase 30b providing a charge of +Q (+I * PW) at electrode E1, and with the first pulse phase 30a providing a charge of +Q and the second pulse phase 30b providing a charge of -Q at the case electrode Ec. In the example shown, such charge balancing is achieved by using the same pulse width (PW) and the same amplitude (|I|) for each of the opposite-polarity pulse phases 30a and 30b. However, the pulse phases 30a and 30b may also be charged balance at each electrode if the product of the amplitude and pulse widths of the two phases 30a and 30b are equal, or if the area under each of the phases is equal, as is known.

Figure 3 shows that stimulation circuitry 28 can include passive recovery switches 41ᵢ, which are described further in U.S. Patent Application Publications 2018/0071527 and 2018/0140831. Passive recovery switches 41ᵢ may be attached to each of the electrode nodes ei 39, and are used to passively recover any charge remaining on the DC-blocking capacitors Ci 38 after issuance of the second pulse phase 30b-i.e., to recover charge without actively driving a current using the DAC circuitry. Passive charge recovery can be prudent, because non-idealities in the stimulation circuitry 28 may lead to pulse phases 30a and 30b that are not perfectly charge balanced.

Therefore, and as shown in Figure 2A, passive charge recovery typically occurs after the issuance of second pulse phases 30b, for example during at least a portion 30c of the quiet periods between the pulses, by closing passive recovery switches 41ᵢ. As shown in Figure 3, the other end of the switches 41ᵢ not coupled to the electrode nodes ei 39 are connected to a common reference voltage, which in this example comprises the voltage of the battery 14, Vbat, although another reference voltage could be used. As explained in the above-cited references, passive charge recovery tends to equilibrate the charge on the DC-blocking capacitors 38 by placing the capacitors in parallel between the reference voltage (Vbat) and the patient's tissue. Note that passive charge recovery is illustrated as small exponentially-decaying curves during 30c in Figure 2A, which may be positive or negative depending on whether pulse phase 30a or 30b have a predominance of charge at a given electrode.

Passive charge recovery 30c may alleviate the need to use biphasic pulses for charge recovery, especially in the DBS context when the amplitudes of currents may be lower, and therefore charge recovery less of a concern. For example, and although not shown in Figure 2A, the pulses provided to the tissue may be monophasic, comprising only a first pulse phase 30a. This may be followed thereafter by passive charge recovery 30c to eliminate any charge build up that occurred during the singular pulses 30a.

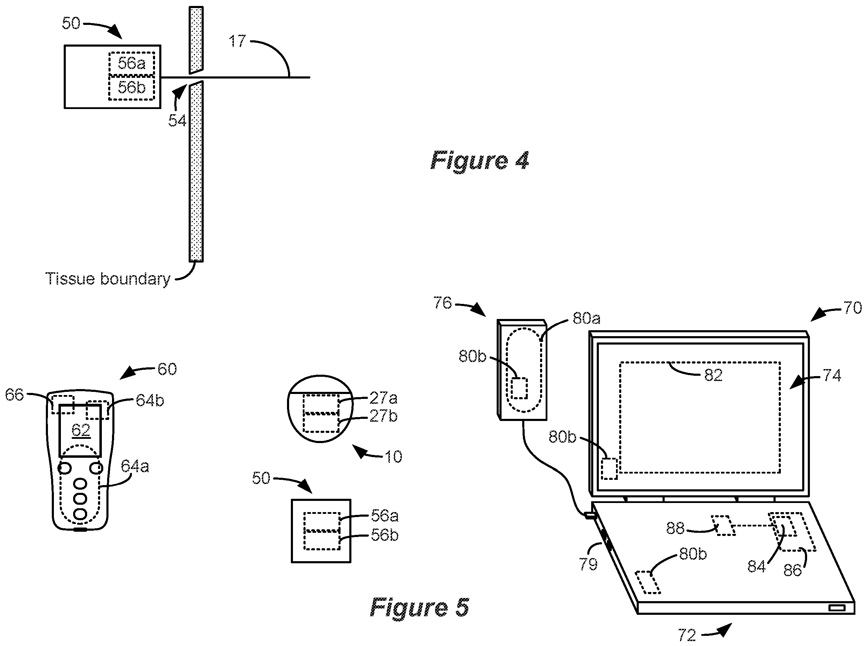

Figure 4 shows an external trial stimulation environment that may precede implantation of an IPG 10 in a patient, for example, during the operating room to test stimulation and confirm the lead position. During external trial stimulation, stimulation can be tried on the implant patient to evaluate side-effect thresholds and confirm that the lead is not too close to structures that cause side effects. Like the IPG 10, the ETS 50 can include one or more antennas to enable bi-directional communications with external devices such as those shown in Figure 5. Such antennas can include a near-field magnetic-induction coil antenna 56a, and/or a far-field RF antenna 56b, as described earlier. ETS 50 may also include stimulation circuitry able to form stimulation in accordance with a stimulation program, which circuitry may be similar to or comprise the same stimulation circuitry 28 (Fig. 3) present in the IPG 10. ETS 50 may also include a battery (not shown) for operational power. The sensing capabilities described herein with regard to the IPG 10, may also be included in the ETS 50 for the purposes described below. Likewise, the ETS may communicate with the CP so that the CP can process the data as described below.

Figure 5 shows various external devices that can wirelessly communicate data with the IPG 10 or ETS 50, including a patient hand-held external controller 60, and a clinician programmer (CP) 70. Both of devices 60 and 70 can be used to wirelessly transmit a stimulation program to the IPG 10 or ETS 50-that is, to program their stimulation circuitries to produce stimulation with a desired amplitude and timing described earlier. Both devices 60 and 70 may also be used to adjust one or more stimulation parameters of a stimulation program that the IPG 10 is currently executing. Devices 60 and 70 may also wirelessly receive information from the IPG 10 or ETS 50, such as various status information, etc.

External controller 60 can be as described in U.S. Patent Application Publication 2015/0080982 for example, and may comprise a controller dedicated to work with the IPG 10 or ETS 50. External controller 60 may also comprise a general-purpose mobile electronics device such as a mobile phone which has been programmed with a Medical Device Application (MDA) allowing it to work as a wireless controller for the IPG 10 or ETS, as described in U.S. Patent Application Publication 2015/0231402. External controller 60 includes a user interface, preferably including means for entering commands (e.g., buttons or selectable graphical elements) and a display 62. The external controller 60's user interface enables a patient to adjust stimulation parameters, although it may have limited functionality when compared to the more-powerful clinician programmer 70, described shortly.

The external controller 60 can have one or more antennas capable of communicating with the IPG 10. For example, the external controller 60 can have a near-field magnetic-induction coil antenna 64a capable of wirelessly communicating with the coil antenna 27a or 56a in the IPG 10 or ETS 50. The external controller 60 can also have a far-field RF antenna 64b capable of wirelessly communicating with the RF antenna 27b or 56b in the IPG 10 or ETS 50.

Clinician programmer 70 is described further in U.S. Patent Application Publication 2015/0360038, and can comprise a computing device 72, such as a desktop, laptop, or notebook computer, a tablet, a mobile smart phone, a Personal Data Assistant (PDA)-type mobile computing device, etc. In Figure 5, computing device 72 is shown as a laptop computer that includes typical computer user interface means such as a screen 74, a mouse, a keyboard, speakers, a stylus, a printer, etc., not all of which are shown for convenience. Also shown in Figure 5 are accessory devices for the clinician programmer 70 that are usually specific to its operation as a stimulation controller, such as a communication "wand" 76 coupleable to suitable ports on the computing device 72, such as USB ports 79 for example.

The antenna used in the clinician programmer 70 to communicate with the IPG 10 or ETS 50 can depend on the type of antennas included in those devices. If the patient's IPG 10 or ETS 50 includes a coil antenna 27a or 56a, wand 76 can likewise include a coil antenna 80a to establish near-field magnetic-induction communications at small distances. In this instance, the wand 76 may be affixed in close proximity to the patient, such as by placing the wand 76 in a belt or holster wearable by the patient and proximate to the patient's IPG 10 or ETS 50. If the IPG 10 or ETS 50 includes an RF antenna 27b or 56b, the wand 76, the computing device 72, or both, can likewise include an RF antenna 80b to establish communication at larger distances. The clinician programmer 70 can also communicate with other devices and networks, such as the Internet, either wirelessly or via a wired link provided at an Ethernet or network port.

To program stimulation programs or parameters for the IPG 10 or ETS 50, the clinician interfaces with a clinician programmer graphical user interface (GUI) 82 provided on the display 74 of the computing device 72. As one skilled in the art understands, the GUI 82 can be rendered by execution of clinician programmer software 84 stored in the computing device 72, which software may be stored in the device's non-volatile memory 86. Execution of the clinician programmer software 84 in the computing device 72 can be facilitated by control circuitry 88 such as one or more microprocessors, microcomputers, FPGAs, DSPs, other digital logic structures, etc., which are capable of executing programs in a computing device, and which may comprise their own memories. For example, control circuitry 88 can comprise an i5 processor manufactured by Intel Corp, as described at https://www.intel.com/ content/ www/ us/ en/ products/ processors/ core/ i5-processors.html. Such control circuitry 88, in addition to executing the clinician programmer software 84 and rendering the GUI 82, can also enable communications via antennas 80a or 80b to communicate stimulation parameters chosen through the GUI 82 to the patient's IPG 10.

The user interface of the external controller 60 may provide similar functionality because the external controller 60 can include the same hardware and software programming as the clinician programmer. For example, the external controller 60 includes control circuitry 66 similar to the control circuitry 88 in the clinician programmer 70, and may similarly be programmed with external controller software stored in device memory.

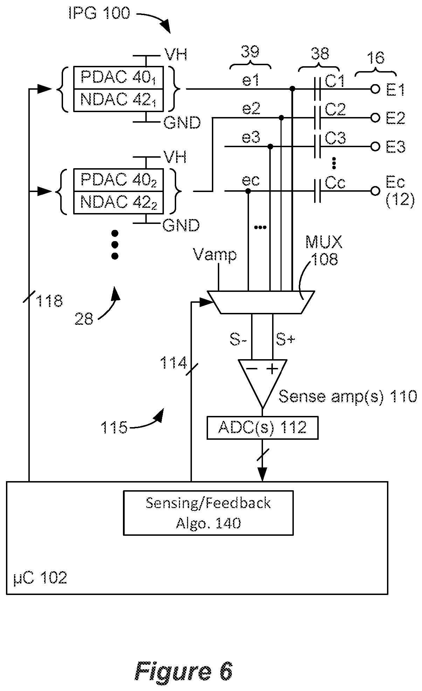

An increasingly interesting development in pulse generator systems is the addition of sensing capability to complement the stimulation that such systems provide. Figure 6 shows an IPG 100 that includes stimulation and sensing functionality. (An ETS as described earlier could also include stimulation and sensing capabilities). Figure 6 shows further details of the circuitry in an IPG 100 that can provide stimulation and sensing innate or evoked signals. The IPG 100 includes control circuitry 102, which may comprise a microcontroller, such as Part Number MSP430, manufactured by Texas Instruments, Inc., which is described in data sheets at http:// www.ti.com/ microcontrollers/ msp430-ultra-low-power-mcus/ overview.html, which are incorporated herein by reference. Other types of controller circuitry may be used in lieu of a microcontroller as well, such as microprocessors, FPGAs, DSPs, or combinations of these, etc. Control circuitry 102 may also be formed in whole or in part in one or more Application Specific Integrated Circuits (ASICs), such as those described earlier

The control circuitry 102 may be configured with one or more sensing/feedback algorithms 140 that are configured to cause the IPG to make certain adjustments and/or take certain actions based on the sensed signal. For example, embodiments of the disclosed IPG are configured to sense evoked neural responses referred to as evoked resonant neural responses (ERNAs), as described below. As explained in more detail below, embodiments of the disclosed IPGs can be configured to adjust or reconfigure stimulation parameters based on the sensed ERNA responses. The algorithms for effecting such adjustments/reconfigurations may be embodied in the sensing/feedback algorithms 140.

The IPG 100 also includes stimulation circuitry 28 to produce stimulation at the electrodes 16, which may comprise the stimulation circuitry 28 shown earlier (Fig. 3). A bus 118 provides digital control signals from the control circuitry 102 to one or more PDACs 40ᵢ or NDACs 42ᵢ to produce currents or voltages of prescribed amplitudes (I) for the stimulation pulses, and with the correct timing (PW, F) at selected electrodes. As noted earlier, the DACs can be powered between a compliance voltage VH and ground. As also noted earlier, but not shown in Figure 4, switch matrices could intervene between the PDACs and the electrode nodes 39, and between the NDACs and the electrode nodes 39, to route their outputs to one or more of the electrodes, including the conductive case electrode 12 (Ec). Control signals for switch matrices, if present, may also be carried by bus 118. Notice that the current paths to the electrodes 16 include the DC-blocking capacitors 38 described earlier, which provide safety by preventing the inadvertent supply of DC current to an electrode and to a patient's tissue. Passive recovery switches 41ᵢ (Fig. 3) could also be present, but are not shown in Figure 6 for simplicity.

IPG 100 also includes sensing circuitry 115, and one or more of the electrodes 16 can be used to sense innate or evoked electrical signals, e.g., biopotentials from the patient's tissue. In this regard, each electrode node 39 is further coupleable to a sense amp circuit 110. Under control by bus 114, a multiplexer 108 can select one or more electrodes to operate as sensing electrodes (S+, S-) by coupling the electrode(s) to the sense amps circuit 110 at a given time, as explained further below. Although only one multiplexer 108 and sense amp circuit 110 are shown in Figure 6, there could be more than one. For example, there can be four multiplexer 108/sense amp circuit 110 pairs each operable within one of four timing channels supported by the IPG 100 to provide stimulation. The sensed signals output by the sense amp circuitry are preferably converted to digital signals by one or more Analog-to-Digital converters (ADC(s)) 112, which may sample the output of the sense amp circuit 110 at 50 kHz for example. The ADC(s) 112 may also reside within the control circuitry 102, particularly if the control circuitry 102 has A/D inputs. Multiplexer 108 can also provide a fixed reference voltage, Vamp, to the sense amp circuit 110, as is useful in a single-ended sensing mode (i.e., to set S- to Vamp).

So as not to bypass the safety provided by the DC-blocking capacitors 38, the inputs to the sense amp circuitry 110 are preferably taken from the electrode nodes 39. However, the DC-blocking capacitors 38 will pass AC signal components (while blocking DC components), and thus AC components within the signals being sensed will still readily be sensed by the sense amp circuitry 110. In other examples, signals may be sensed directly at the electrodes 16 without passage through intervening capacitors 38.

According to some embodiments, in may be preferred to sense signals differentially, and in this regard, the sense amp circuitry 110 comprises a differential amplifier receiving the sensed signal S+ (e.g., E3) at its non-inverting input and the sensing reference S- (e.g., E1) at its inverting input. As one skilled in the art understands, the differential amplifier will subtract S- from S+ at its output, and so will cancel out any common mode voltage from both inputs. This can be useful for example when sensing various neural signals, as it may be useful to subtract the relatively large-scale stimulation artifact from the measurement (as much as possible). Examples of sense amp circuitry 110, and manner in which such circuitry can be used, can be found in U.S. Patent Application Publication 2019/0299006; and U.S. Provisional Patent Application Serial Nos. 62/825,981, filed March 29, 2019; 62/825,982, filed March 29, 2019; and 62/883,452, filed August 6, 2019.

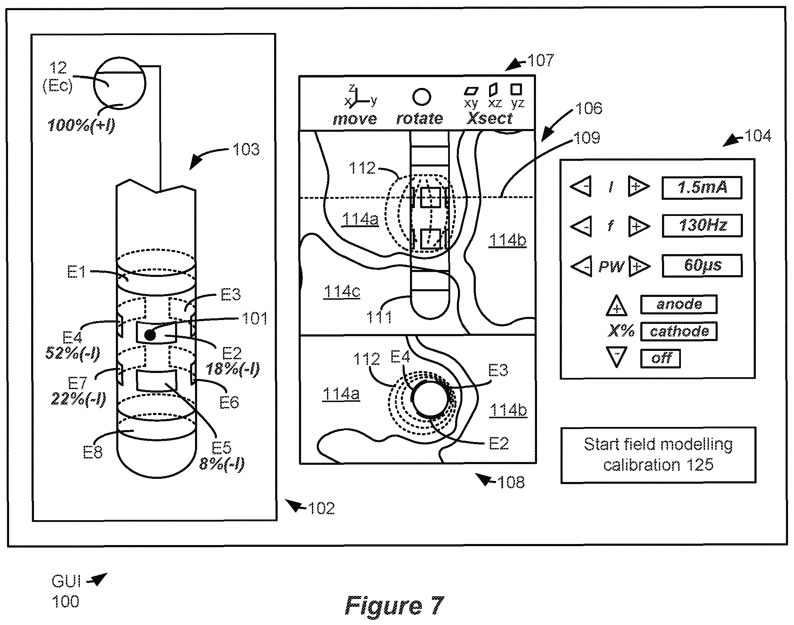

Particularly in the DBS context, it can be useful to provide a clinician with a visual indication of how stimulation selected for a patient will interact with the tissue in which the electrodes are implanted. This is illustrated in Figure 7, which shows a Graphical User Interface (GUI) 100 operable on an external device capable of communicating with an IPG 110 or ETS 150. Typically, and as assumed in the description that follows, GUI 100 would be rendered on a clinician programmer 70 (Fig. 5), which may be used during surgical implantation of the leads, or after implantation when a therapeutically useful stimulation program is being chosen for a patient. However, GUI 100 could be rendered on a patient external programmer 60 (Fig. 5) or any other external device capable of communicating with the IPG 110 or ETS 150.

GUI 100 allows a clinician (or patient) to select the stimulation program that the IPG 110 or ETS 150 will provide and provides options that control sensing of innate or evoked responses, as described below. In this regard, the GUI 100 may include a stimulation parameter interface 104 where various aspects of the stimulation program can be selected or adjusted. For example, interface 104 allows a user to select the amplitude (e.g., a current I) for stimulation; the frequency (f) of stimulation pulses; and the pulse width (PW) of the stimulation pulses. Stimulation parameter interface 104 can be significantly more complicated, particularly if the IPG 100 or ETS 150 supports the provision of stimulation that is more complicated than a repeating sequence of pulses. See, e.g., U.S. Patent Application Publication 2018/0071513. Nonetheless, interface 104 is simply shown for simplicity in Figure 7 as allowing only for amplitude, frequency, and pulse width adjustment. Stimulation parameter interface 104 may include inputs to allow a user to select whether stimulation will be provided using biphasic (Fig. 2A) or monophasic pulses, and to select whether passive charge recovery will be used, although again these details aren't shown for simplicity.

Stimulation parameter interface 104 may further allow a user to select the active electrodes-i.e., the electrodes that will receive the prescribed pulses. Selection of the active electrodes can occur in conjunction with a leads interface 102, which can include an image 103 of the one or more leads that have been implanted in the patient. Although not shown, the leads interface 102 can include a selection to access a library of relevant images 103 of the types of leads that may be implanted in different patients.

In the example shown in Figure 7, the leads interface 102 shows an image 103 of a single split-ring lead 33 similar to that described earlier with respect to Figure 1B. The leads interface 102 can include a cursor 101 that the user can move (e.g., using a mouse connected to the clinician programmer 70) to select an illustrated electrode 16 (e.g., E1-E8, or the case electrode Ec). Once an electrode has been selected, the stimulation parameter interface 104 can be used to designate the selected electrode as an anode that will source current to the tissue, or as a cathode that will sink current from the tissue. Further, the stimulation parameter interface 104 allows the amount of the total anodic or cathodic current +I or -I that each selected electrode will receive to be specified in terms of a percentage, X. For example, in Figure 7, the case electrode 12 Ec is specified to receive X=100% of the current I as an anodic current +I. The corresponding cathodic current -I is split between electrodes E2 (0.18*-I), E4 (0.52*-I), E5 (0.08*-I), and E7 (0.22*-I). Thus, two or more electrodes can be chosen to act as anodes or cathodes at a given time using MICC (as described above), allowing the electric field in the tissue to be shaped. The currents so specified at the selected electrodes can be those provided during a first pulse phase (if biphasic pulses are used), or during an only pulse phase (if monophasic pulses are used).

GUI 100 can further include a visualization interface 106 that can allow a user to view an indication of the effects of stimulation, such as electric field image 112 formed on the one or more leads given the selected stimulation parameters. The electric field image 112 is formed by field modelling in the clinician programmer 70. Only one lead is shown in the visualization interface 106 for simplicity, although again a given patient might be implanted with more than one lead. Visualization interface 106 provides an image 111 of the lead(s) which may be three-dimensional.

The visualization interface 106 preferably, but not necessarily, further includes tissue imaging information 114 taken from the patient, represented as three different tissue structures 114a, 114b and 114c in Figure 7 for the patient in question, which tissue structures may comprise different areas of the brain for example. Such tissue imaging information may comprise a Magnetic Resonance Image (MRI), a Computed Tomography (CT) image or other type of image, and is preferably taken prior to implantation of the lead(s) in the patient. Often, one or more images, such as an MRI, CT, and/or a brain atlas are scaled and combined in a single image model. As one skilled in the art will understand, the location of the lead(s) can be precisely referenced to the tissue structures 114i because the lead(s) are implanted using a stereotactic frame (not shown). This allows the clinician programmer 70 on which GUI 100 is rendered to overlay the lead image 111 and the electric field image 112 with the tissue imaging information in the visualization interface 106 so that the position of the electric field 112 relative to the various tissue structures 114i can be visualized. The image of the patient's tissue may also be taken after implantation of the lead(s), or tissue imaging information may comprise a generic image pulled from a library which is not specific to the patient in question.

The various images shown in the visualization interface 106 (i.e., the lead image 111, the electric field image 112, and the tissue structures 114i) can be three-dimensional in nature, and hence may be rendered in the visualization interface 106 in a manner to allow such three-dimensionality to be better appreciated by the user, such as by shading or coloring the images, etc. Additionally, a view adjustment interface 107 may allow the user to move or rotate the images, using cursor 101 for example.

GUI 100 can further include a cross-section interface 108 to allow the various images to be seen in a two-dimensional cross section. Specifically, cross-section interface 108 shows a particular cross section 109 taken perpendicularly to the lead image 111 and through split-ring electrodes E2, E3, and E4. This cross section 109 can also be shown in the visualization interface 106, and the view adjustment interface 107 can include controls to allow the user to specify the plane of the cross section 109 (e.g., in XY, XZ, or YZ planes) and to move its location in the image. Once the location and orientation of the cross section 109 is defined, the cross-section interface 108 can show additional details. For example, the electric field image 112 can show equipotential lines allowing the user to get a sense of the strength and reach of the electric field at different locations. Although GUI 100 includes stimulation definition (102, 104) and imaging (108, 106) in a single screen of the GUI, these aspects can also be separated as part of the GUI 100 and made accessible through various menu selections, etc.

It has been observed that DBS stimulation in certain positions in the brain can evoke resonant neural responses, referred to herein as evoked resonant neural responses (ERNAs). See, e.g., Sinclair, et al., "Subthalamic Nucleus Deep Brain Stimulation Evokes Resonant Neural Activity," Ann. Neurol. 83(5), 1027-31, 2018. ERNA responses are observed when a first, excited neural population excites a second neural population, which in tum, re-excites the first neural population. The ERNA responses typically have an oscillation frequency of about 200 to about 500 Hz. Stimulation of the STN, and particularly of the dorsal subregion of the STN, has been observed to evoke strong ERNA responses, whereas stimulation of the posterior subthalamic area (PSA) does not evoke such responses. *Id.* Thus, ERNA can provide a biomarker for electrode location, which can potentially indicate acceptable or perhaps optimal lead placement and/or stimulation field placement for achieving the desired therapeutic response.

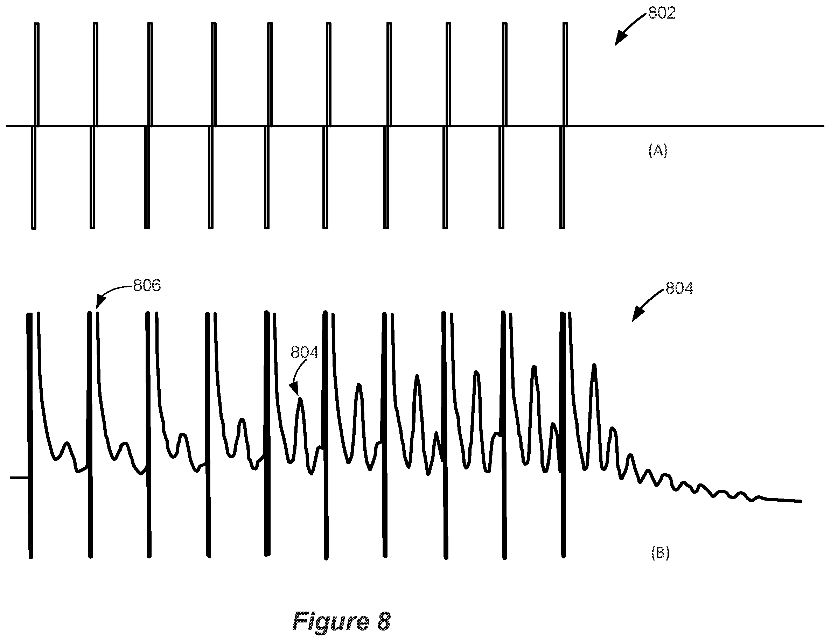

Figure 8 illustrates an example of an ERNA response to a burst of ten symmetric monopolar biphasic pulses 802. The stimulation waveform 802 is illustrated as trace (A) and the measured response 804 is illustrated as trace (B). The measured response comprises stimulation artifacts 806 immediately following each stimulation pulse. The measured response may also include an evoked compound action potential (ECAP) occurring 1-2 milliseconds after the stimulation pulse, though the ECAP in trace (B) is obscured by the stimulation artifact. The ERNA responses 804 build following each pulse. Notice that the amplitude of the oscillating ERNA response 804 increases during the burst and then persists even after the stimulation ceases. In the illustrated embodiment, stimulation and recording is provided at the same electrodes, but different electrodes may be used for providing stimulation and recording.

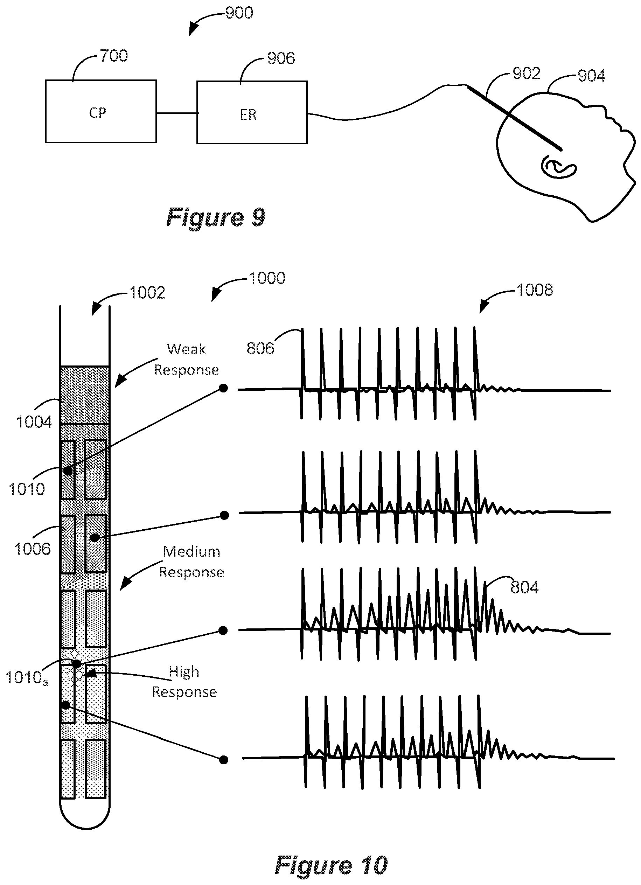

Aspects of the disclosure provide methods and systems for using ERNA responses sensed during stimulation to direct lead placement during lead implantation surgery. Figure 9 illustrates a schematic of a system 900 for performing implantation of an electrode lead 902 in the brain of a patient 904. The electrode lead may generally be any lead configured for DBS, but preferably comprises 8 or more electrodes. More electrodes on the lead can provide greater resolution. The electrode lead may comprise directional electrodes (such as the electrodes illustrated on lead 33 (Fig. 1B)). Greater resolution is also provided by the ability to use MICC and current steering to provide stimulation locations that may be between the locations of actual physical electrodes. Thus, the system 900 provides the ability to fine tune the stimulation location with a high degree of precision to find a stimulation location that provides the best ERNA response.

The system 900 also comprises one or more devices for controlling the stimulation and sensing provided at the electrode lead 902. The illustrated embodiment comprises a clinician programmer (CP) 700 for programming the stimulation and sensing parameters. The functionality of a CP 700 may be similar to that described above with respect to CP 70 (FIG. 5), for example. Note that the call-out number 700 is used to refer to the CP of the system 900, whereas the call-out number 70 is used in FIG. 5, simply to denote that CP 700 is used in the operating room during electrode lead implantation, whereas CP 70 is used after implantation to program the patient's IPG. However, both CP 700 and CP 70 may have similar functionality and may actually be the same device. The clinician can use the CP 700 to select the electrodes of the lead 902 that will be used to provide stimulation, the parameters of the stimulation waveform(s) that will be applied, and the electrode(s) that will be used to sense evoked responses. In the illustrated system 900, the CP 700 provides those selections to an evoked response stimulation and recording programmer (ER) 906. The ER 906 causes the stimulation to be applied to at lead the 902. The ER 906 also receives, and records sensed signals from the lead. The CP and ER may communicate via a wired or a wireless connection. In the illustrated embodiment, a single ER 906 component is shown. However, according to some embodiments, multiple components could be used, for example, separate components for providing stimulation and for receiving and recording sensed signals. The CP may communicate with either or both of the ER components in such an embodiment. According to some embodiments, aspects of the CP functionality and the ER functionality may be combined in a single device. For example, the ER 906 may itself be configured for programming the stimulation and/or sensing parameters. Alternatively, the functionality of receiving and recording the sensed signals (correlated with the stimulation configuration/parameters) may be embodied in the CP 700, for example as a module or subroutine additional to the CP functionality described above. Regardless of the exact configuration, the system is capable of causing stimulation of a defined waveform to be applied using selected one or more electrode on the lead, and of sensing/recording responses evoked by the stimulation. Further, the system 900 (e.g., in either the CP 700 or the ER 906) comprises control circuitry configured to perform the steps of the various algorithms and methods that are described below.

According to some embodiments, the system 900 (e.g., in either the CP 700 or the ER 906) is configured to process the sensed signals evoked by the stimulation. The system may include a user interface configured to display an indication of the sensed response data in relation to the stimulation parameters and configurations. Figure 10 illustrates aspects of an embodiment of such a user interface (UI) 1000. The illustrated UI includes a representation 1002 of the lead being implanted. In the illustrated example, the lead comprises 16 electrodes, including a single ring electrode 1004 and 15 segmented electrodes 1006. Examples of such leads and other suitable leads are described in U.S. Patent No. 10,286,205.

The UI 1000 also includes representations 1008 of the sensed signals. As explained with reference to Fig. 8 above, the sensed signals may comprise a stimulation artifact component 806 and an ERNA component 804. The system may be configured to display a representation of correlations between the sensed signals and the stimulation parameters and/or configurations. For example, in the illustrated UI, stimulation was applied sequentially at the locations on the lead indicated by the black circles 1010. For each of the stimulation locations, a corresponding representation of the sensed data is displayed.

According to some embodiments, the system may be configured with algorithms to sequentially apply stimulation at various positions upon the lead and to optimize the stimulation location to provide the best ERNA response. It should be appreciated that the stimulation locations on the lead may or may not correspond to positions of physical electrodes. For example, MICC can be used, as described above, to provide stimulation at locations that do not directly coincide with physical electrodes, such as location 1010ₐ. The availability of MICC to provide stimulation at any location on the lead provides high resolution for locating a stimulation location with a maximum ERNA response. Various parameters or waveform characteristics extracted from the ERNA response may be used to optimize the stimulation location. Example parameters or waveform characteristics include amplitude, rate of decay, number of pulses, frequency, and the like.

According to some embodiments, the UI 1000 displays raw data (e.g., signal traces) of the sensed signals correlated to the various stimulation locations, as illustrated in Figure 10. Thus, the ERNA responses evoked at the various stimulation locations can be visually compared. According to some embodiments, the raw measured sensed data may be processed to provide further information that may be displayed by the UI 1000. For example, in Figure 10, the representation 1002 of the electrode lead is overlayed with a false-color map (i.e., a color-coded map) that correlates the ERNA response to stimulation positions upon the lead. For example, the colors on the false-color map may indicate areas of the electrodes where stimulation results in weak, medium, or strong ERNA responses. Additionally (or alternatively) the UI may associate numerical values with positions upon the representation of the lead indicating the intensity of ERNA responses caused by stimulation at those positions.

According to some embodiments, the UI may provide clinical decision support for the implantation team or clinician. For example, the UI may provide an indication of the likelihood that an electrode lead, in its present position, is likely to provide therapy that is good and robust. Such information is useful to inform the clinician's decision to leave the lead in its present location or to seek a better location. Such an indication may be based on historical data correlating one or more parameters of measured ERNA responses with therapeutic efficacy. Such historical data may be configured within a database, for example. If the extent of historic data is adequate, then the system may provide a quantitative prediction of efficacy. For example, the indication might provide a numerical value (such as percentage value) that the present location of the lead will provide good therapy. Alternatively, the system may provide a binary (yes/no) indication of whether the lead placement is expected to provide high efficacy. The determination of whether the lead placement is satisfactory may be based on a threshold value for the ERNA response derived from the data base, for example.

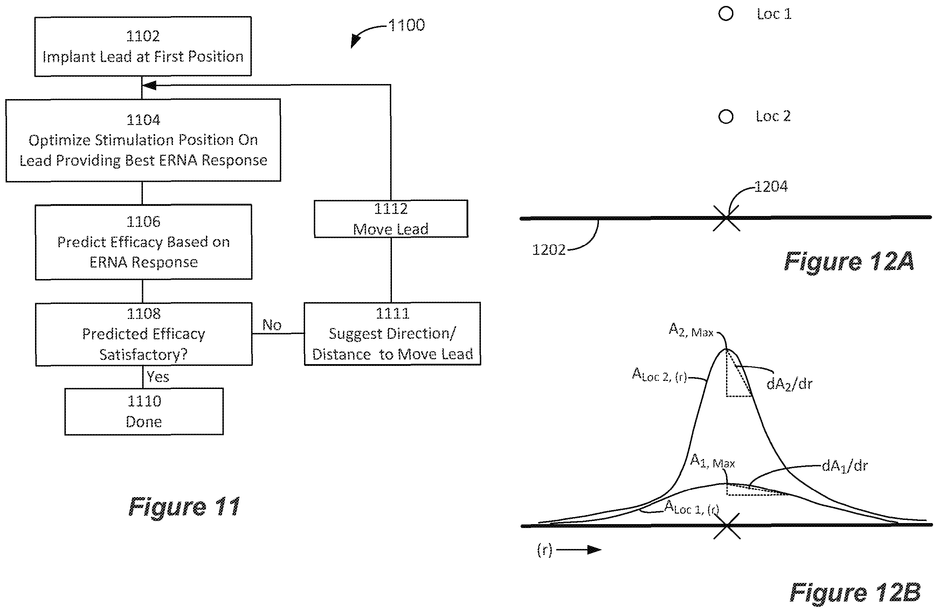

Figure 11 illustrates a workflow 1100 for using measured ERNA responses for directing the implantation of a lead. The steps of the workflow 1100 may be performed using control circuitry of the system 900, for example, control circuitry of the CP 700 and/or the ER 906). Assume that the lead has been implanted at a first position (Step 1102). The control circuitry may receive an indication that the lead is positioned at the first position. The indication may simply be the clinician initiating the workflow. At Step 1104 an optimization algorithm is used to determine the position on the lead where stimulation provides the best ERNA response. As explained above, MICC may be used to stimulate at positions that do not correspond to physical electrodes. Once the best stimulation position on the lead is determined, the algorithm predicts the efficacy that can be expected for therapy using the present lead position (Step 1106). At Step 1108 it is determined if the predicted efficacy at the present lead location is adequate. If the answer is yes, then the position optimization is complete (Step 1110). If the predicted efficacy is not adequate, then the algorithm may suggest a distance/direction to move the lead (Step 1111) and lead may be repositioned (Step 1112) and the optimization routines may be performed again.

According to some embodiments, the system 900 may provide further surgical support during lead implantation by providing information relating to the lead location with respect to the neural target (i.e., the neural element(s) generating the ERNA response). For example, the system may use source localization techniques to calculate the distance and the direction of the target from the lead, and provide such estimates to a clinician. The clinician may use that information to determine how to move the lead, if necessary. For example, the clinician may decide to remove the lead and re-implant it on a parallel track some distance from the original track or the clinician may opt to change the lead trajectory.

According to some embodiments, the direction and/or distance from the lead to the ERNA source may be determined based on the measured amplitude of the ERNA response. For example, a model can be developed based on clinical data that uses amplitude data to estimate the distance between the trajectory of the electrode lead and the neural source of the ERNA signal. Figure 12 A illustrates an electrode lead 1202, which may comprise a plurality of electrodes configured according to any of the embodiments described above. Assume that the neural target (i.e., the ERNA source) is located at either Loc 1 or Loc 2, which are different distances from the lead 1202. It would be expected that the if the ERNA source is at Loc 2, the amplitude of the sensed ERNA response would be greater than if the ERNA source is at the more distant Loc 1. According to some embodiments, a stimulation optimization routine using MICC, as described above, can be used to determine the stimulation position on the lead 1204 which evokes the greatest ERNA amplitude. The maximum ERNA response may be used for source localization. Likewise, the variation in ERNA response to stimulation at various positions upon the electrode lead may be used for source localization. Figure 12B shows the ERNA amplitude as a function of stimulation location (r) along the electrode lead for locations Loc 1 and Loc 2. Notice that the maximum amplitude (A_{2, Max}) measured for the near location Loc 2 is greater than the maximum amplitude (A_{3, Max}) measured for the far location Loc 1. Also notice that the slopes (i.e., the derivatives) of the amplitudes, dA₂/dr and dA₁/dr, are different. Generally, for a location (such as Loc 2) that closer to the lead, the slope of the amplitude curve is greater than the slope of the amplitude curve for a more distant ERNA source (like Loc 1). Thus, a model for source location using measured ERNA responses may be based on the maximum amplitude of the ERNA response and on the slope of the curve of the amplitude as a function of position on the electrode lead. Multiple values for those properties may be determined at different slices in time. The model may also consider other aspects, such as the patient's medication state.

According to some embodiments, the source localization may be based on a modeling technique such as an inverse solution approximation. The inverse solution approximation involves a transformation matrix that converts electrical signals into localized electrical activity confined to a solution space, representing the volume and shape of the brain or an appropriate subset of brain regions. Localization is accomplished by multiplication of each new ERNA measurement by a transformation matrix (T) that is generated by the inverse solution approximation algorithm in use to yield the array of voxels containing localized electrical activity (V). Voxels are defined as discrete units of volume within the solution space and they contain the localized electrical activity for that particular region of the brain. The localized electrical activity for each voxel is represented as a three-dimensional vector with an x, y and z component. Essentially, the source localization algorithm seeks to minimize error between a plurality of potential source locations and the measured data, for example, by using a least-squares fit. Examples of suitable source localization algorithms include s-LORETA, which is described in R. D. Pascual-Marqui, "Standardized low-resolution brain electromagnetic tomography (sLORETA): technical details, Methods & Findings in Experimental & Clinical Pharmacology, 24D, 2002, 5-12. Other examples include the locally optimal source (LOS) method described in Laarne, et al., "Accuracy of two dipolar inverse algorithms applying reciprocity for forward calculation," Comput. Biomed. Res., 33, 2000, 172-185. Other source localization methods are known in the art and may be used.

Thus, according to some embodiments, the system may use a source localization technique, as described above, to determine the relative position of the electrode lead with respect to the ERNA response. The UI of the system, such as UI 1000 (FIG. 10), may present information relating to the location of the ERNA source and/or feedback regarding how to move the electrode lead. For example, the UI may provide a distance and direction to the target. It should be appreciated that the optimal lead placement may, or may not, correspond to the location of the maximum ERNA response. But in either case, the ERNA source may be an indicator, or signpost, for the optimum therapeutic lead placement. For example, perhaps the maximum ERNA location is consistently 1 mm inferior to the optimal stimulation location. Then the algorithms may use this known relationship to provide estimates of the optimal location to the clinician.

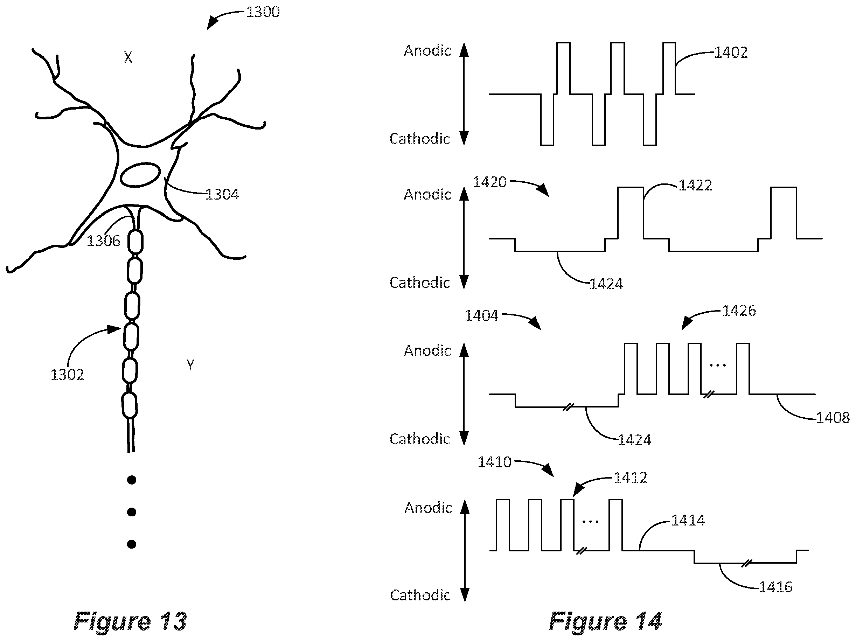

According to some embodiments, the placement of the electrode lead with respect to a neural target (i.e., an ERNA source) may be determined based on variations of ERNA responses evoked by cathodic v. anodic stimulation. Such methods take advantage of the fact that anodic and cathodic current stimulate different locations of neurons at different threshold currents. Referring to the schematic of a neuron 1300 illustrated in Figure 13, an electrode placed at a position Y, near an axon of passage 1302, will stimulate the axon at a lower amplitude using cathodic current than is required to stimulate the axon using anodic current. In contrast, an electrode at position X, near the cell body 1304 and with the axon 1302 travelling away from the electrode, will stimulate the axon hillock 1306 near the cell body at a lower amplitude using anodic current than using cathodic current. The neuron terminals and soma are recruited in a different order with cathodic stimulation than they are with anodic stimulation. Thus, sensing the ERNA responses evoked by cathodic stimulation and the ERNA responses evoked by anodic stimulation can provide information relating the position of the lead relative to the neural target.

Since the recruitment order using anodic and cathodic stimulation is sensitive to the orientation of the electrodes with respect to the neural elements, determining the ERNA responses using both anodic and cathodic stimulation can help the physician determine if the trajectory angle of the lead implantation is correct and/or if the lead needs to be moved. For example, assume that the axons of a population of neurons, such as the neuron 1300, is the target for stimulation. Those axons could be stimulated using either cathodic or anodic stimulation. If the electrodes are near the cell body 1304 (i.e., at position X), then lower amplitude anodic stimulation could be used. If the electrodes are near the axon 1302 (i.e., at position Y), then lower amplitude cathodic stimulation could be used. Now assume that, for clinical reasons, the clinician wishes to stimulate a certain population of axons with near-by cell bodies but to avoid stimulating a second population of axons that do not have near-by cell bodies. In that case, the clinician may opt to use anodic stimulation to preferentially stimulate the axons with near-by cell bodies. To determine the proper electrode placement, the ERNA responses to both anodic and cathodic stimulation can be compared. If the anodic stimulation evokes a greater ERNA response, that suggests that the electrode placement is appropriate to stimulate axons with near-by cell bodies (i.e., the clinician's goal). If the cathodic stimulation evokes a greater ERNA response, that suggests that the clinician should change the trajectory of the electrode lead.

As described above, a system, such as system 900 (FIG. 9) can provide surgical support by providing information to the clinician concerning lead placement and the location upon the lead that evokes favorable ERNA responses. Those determinations may be made with a high degree of special precision using leads with high number of electrodes (e.g., > 8 electrodes) and by using MICC.

As mentioned above, once the electrode lead(s) have been implanted in the patient and the patient is configured with an IPG 10 (or ETS 50) (FIG. 5), the patient will typically undergo a fitting procedure during which their IPG is programmed with optimal stimulation parameters for treating their symptoms. That fitting procedure is typically performed using a CP 70 (FIG. 5), which may include one or more UIs 100 (FIG. 7). The ERNA response may be used as a biomarker to help direct the programming of the therapeutic stimulation. According to some embodiments, the ERNA determinations from the lead placement operation can be preserved and used for the subsequent programming of the stimulation parameters for the patient's IPG. According to some embodiments, the ERNA data collected during lead implantation may be stored in the CP 700 (FIG. 9) and the programming clinician may use the same CP 700 (instead of CP 70) for programming the stimulator during the subsequent fitting procedure. Thus, the programming clinician may have access to the full raw operating room ERNA data and/or the processed ERNA data (i.e., the false color map(s), the ERNA parameter values, etc.). Alternatively, the ERNA data (raw and/or processed) may be transferred to a separate programmer (e.g., CP 70, FIG. 5), either wirelessly (e.g., via internal servers, electronic medical record (EMR) systems, the cloud, etc.) or using a storage device (e.g., USB drive or other drive). According to some embodiments, the programming system that the programming clinician uses to program the IPG stimulation parameters may be configured to automatically load the stimulation parameters (e.g., the MICC settings) that yielded the best ERNA response in the operating room.

According to some embodiments, the CP 70 may be configured to display ERNA responses to stimulation parameters programmed during the fitting process. For example, CP 70 may display a UI 1000 (FIG. 10) as described above. Thus, the fitting clinician may further refine the MICC parameters based on the sensed ERNA responses to further refine the stimulation location on the electrode lead. A further aspect of the fitting process may be deriving pulse shapes, polarities, and waveforms for maximizing the amount of information provided by the sensed ERNA responses.

According to some embodiments, ERNA responses may be used both during the fitting process and during ongoing therapy as a biomarker or indicator of effective stimulation and/or of patient state. For example, the patient's IPG may be configured with one or more algorithms to determine ERNA responses to stimulation. For example, the algorithms may be configured as part of the sensing/feedback algorithm 140 executable in the control circuitry 102 of the IPG 10 (FIG. 6). Thus, according to some aspects of the disclosure, the fitting process may address two considerations: (1) determining the optimum stimulation parameters for providing therapy to the patient, and (2) determining optimum parameters for evoking ERNA responses for use as a biomarker/feedback variable.

An aspect of determining optimum parameters for evoking and sensing ERNA responses may comprise determining a waveform that evokes a strong ERNA response. Referring again to Figure 8, the illustrated ERNA response 804 was evoked using a symmetric biphasic pulse 802. But as explained above, different pulse shapes and pulse polarities have different effects on neural elements, for example, effecting the order in which neural elements are recruited, and in some cases, symmetric biphasic stimulation waveforms may not be the most suitable waveforms for providing therapy and/or for evoking the strongest ERNA response. Thus, during the fitting process, various waveform shapes may be explored.

Figure 14 illustrates various waveforms. Waveform 1402 is a symmetric biphasic pulse similar to the waveform 802 that evoked the ERNA response 804 (FIG. 8). With such symmetric biphasic pulses cathodic and anodic effects on neural elements and on ERNA responses may be combined. Recently, anodic waveforms have been explored for DBS. For example, waveform 1420 includes a strong anodic phase 1422 preceded by a low-amplitude cathodic phase 1424. The evoked effect is primarily driven by the higher amplitude anodic phase 1422, yielding an effect predominantly from a single polarity. The preceding low-amplitude phase 1424 serves to charge-balance the waveform and can enhance the effect of the high-amplitude phase.

To separate anodic from cathodic effects, monopolar waveforms, such as waveform 1404 may be used. Waveform 1404 comprises a low-amplitude cathodic phase 1424, a series of anodic pulses 1426, and a quiescent period 1408. The low-amplitude cathodic phase has an amplitude that is preferably below a threshold value required to recruit neural elements, so it has little or no stimulatory effect. The length of the phase preferably is sufficient to pass enough cathodic charge to balance the anodic charge passed during the anodic pulses 1426. The ERNA response may be sensed during the quiescent phase 1408. Using a waveform such as the waveform 1404 allows the ERNA response evoked by the anodic stimulation to be measured without interference from cathodic effects. Likewise, the polarity of each of the phases could be inverted to form a charge-balance waveform of cathodic pulses for measuring ERNA responses evoked using cathodic stimulation. Waveform 1410 is another example of a waveform that can allow the ERNA response evoked by anodic stimulation to be measured without interference from cathodic effects. Waveform 1410 comprises a series of anodic pulses 1412 followed by a quiescent phase 1414, which is followed by a charge recovery phase 1416. The charge recovery period 1416 may comprise low-amplitude active charge recovery and/or passive charge recovery, as is known in the art. The ERNA response may be sensed during the quiescent phase 1414. Again, the polarity of each of the phases could be inverted to form a charge-balance waveform of cathodic pulses for measuring ERNA responses evoked using cathodic stimulation.

Figure 14 illustrated examples of waveforms (e.g., waveforms 1404 and 1410) that can be particularly useful for evoking and sensing ERNA responses. As explained, those waveforms may contain a series of pulses (e.g., monopolar pulses), followed by a quiescent phase during which the ERNA responses are detected. According to some embodiments, the quiescent period is 4 milliseconds (ms) or longer, for example, 10 ms, 20 ms, or 30 ms. The waveforms may include a passive and/or active charge recovery phase as well. It should be noted that the ideal waveform for detecting ERNA responses may or may not also be the ideal waveform for providing therapy. In other words, according to some embodiments a first waveform (referred to herein as a therapy waveform) may be used for providing therapeutic relief of a patient's symptoms and a second waveform (referred to herein as an interrogation waveform) may be used to evoke an ERNA response that can be used as a biomarker and/or feedback variable. For example, a biphasic waveform such as waveform 1402 (FIG. 14) or a monophasic waveform such as waveform 1420 (FIG. 22, either anodic or cathodic) may be ideal for providing therapy, but those waveforms may not be ideal for evoking/detecting ERNA response, whereas pulsed waveforms, such as waveforms 1404 and/or 1410 may be particularly suited for evoking/detecting ERNA response. It should also be noted that the stimulating electrodes/positions used to apply the interrogation waveform may different than the stimulating electrodes/positions used to provide the therapeutic stimulation. According to some embodiments, the therapy waveform and the interrogation waveform may be provided by different electrode leads located in different neural tissues.

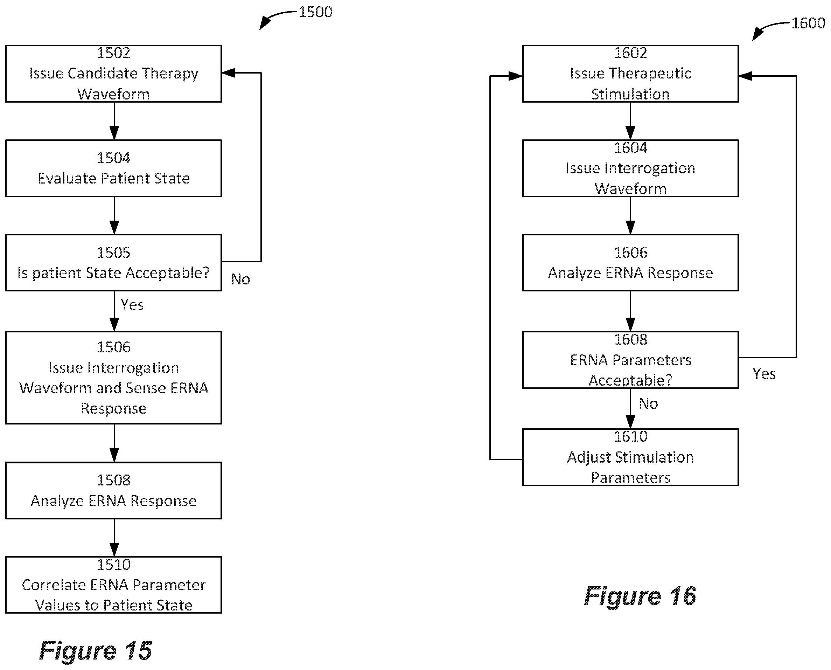

Thus, the fitting process may involve determining interrogation waveforms/locations as well as determining therapy waveforms/locations. For example, the clinician may try different candidate interrogation waveforms to determine which interrogation waveform allows the best sensing of ERNA responses. Once the best interrogation waveform has been determined, the interrogation waveform may be used during the fitting process, for example, as shown in Figure 15. In the illustrated fitting process 1500, a candidate therapy waveform is applied (Step 1502) and the patient's state in response to the candidate therapy waveform can be evaluated (Step 1504). For example, the stimulation using the candidate therapy waveform may result in a decrease in the patient's tremor or result in some other therapeutic effect. At step 1505, it can be determined if the patient state is acceptable. In other words, it is determined if the candidate therapy waveform is suitable for providing therapy to the patient. If the patient state is not ideal, then a further candidate waveform can be tried. Steps 1502 through 1505 can be iteratively repeated to determine one or more candidate waveforms (and stimulation locations) that provide the best improvement in the patient's symptoms. Once an acceptable patient state is achieved, at step 1506, the interrogation waveform (as previously determined, as described above) can be issued to evoke an ERNA response. At step 1508, parameters of the ERNA response can be analyzed. As mentioned above, example ERNA parameters that may be determined include amplitude, rate of decay, frequency, and the like. At step 1510, the analyzed parameters of the ERNA responses can be correlated to the determined patient state. In sum, the fitting procedure 1500 determines 1) therapeutic stimulation parameters that are suitable for providing therapy for a patient, and 2) correlations between the patient's state and one or more parameters of ERNA responses evoked by the interrogation waveform.

During the patient's ongoing therapy, the correlations between the patient's state and the determined one or more parameters of ERNA responses can be used as biomarkers for closed-loop feedback to adjust the patient's therapy, for example, as illustrated in Figure 16. During the closed-loop feedback workflow 1600 the patient's IPG provides therapeutic stimulation 1602 according to one of the stimulation programs determined during the fitting process. Periodically during the provision of the therapeutic stimulation, the IPG issues an interrogation waveform to evoke ERNA responses (Step 1604). For example, the therapeutic stimulation may be interrupted, and the interrogation waveform may be issued. Alternatively, the interrogation waveform may be issued while therapeutic stimulation is occurring. At step 1606, the ERNA algorithm (e.g., the sensing/feedback algorithm 140 of the IPGs microcontroller, FIG. 6) analyzes the ERNA response to extract one or more ERNA parameters. At step 1608, the ERNA algorithm determines if the ERNA parameter(s) are commensurate with a desirable patient state (as determined during the fitting procedure 1500, FIG. 15). If the ERNA parameters are commensurate with a desirable patient state then the system continues applying the therapeutic stimulation. If the ERNA parameters are not commensurate with a desirable patient state, then the ERNA algorithm may adjust the stimulation based on the ERNA parameters. According to some embodiments, the ERNA algorithm may comprise one or more control models that seek to adjust the stimulation in a manner that causes the monitored neural elements to express ERNA responses that were determined to correlate with effective therapy. The control models may be essentially any algorithm that is configured to adjust the stimulation settings to minimize the difference between the measured ERNA features and the desired ERNA features. Examples of control models include such algorithms as Kalman filter algorithms, heuristic control algorithms, simple threshold control model, and proportional-integral-derivative (PID) controller models. The control model may also be a hybrid of any of these methods. Thus, the ERNA algorithm can provide closed-loop feedback control of the stimulation based on the sensed ERNA parameters. The parameters may be adjusted iteratively, as illustrated in Figure 16, to maintain appropriate therapy.

In some diseases treatable using DBS, like Parkinson's disease, the patient may experience "fluctuations" in patient state that are largely due to the use of medication that washes in upon taking it, and then washes out over time. Medication and stimulation may interact, such that less stimulation is needed when the medication is in the patient's body and more stimulation is needed when the medication is not in the patient's body. According to some embodiments, the IPG can be configured to evaluate ERNA parameter responses to estimate the medication state of the patient. Medication wash-in times are typically on the order of tens of minutes to hours. Thus, according to some embodiments, ERNA evaluations may be performed at a frequency of minutes or tens of seconds, which allows averaging many samples to reduce noise. Once the medication state of the patient is estimated, stimulation can be adjusted accordingly, to a predicted optimal stimulation for the patient given the medication state. According to some embodiments, stimulation change boundaries may be set by the clinician and/or the ERNA algorithm to prevent step changes in the stimulation beyond a prescribed amount. According to some embodiments, the patient may be allowed to manually make adjustments that exceed the step boundary limitations using their external controller. According to some embodiments, a medication calibration fitting session may be performed to calibrate one or more ERNA features (i.e., amplitude, rate of decay, frequency, and the like) with the patient's medication state. For example, ERNA measurements may be taken in the absence of medication to "learn" the patient-specific off-medication ERNA signature. The clinician may then administer medication and obtain ERNA measurements as the medication washes in, is at steady state, and washes out. The observed calibration measurements can then be used during ongoing therapy to determine the patient's medication state.

The present invention is set out in the claims that follow.

## Claims

1. An apparatus for facilitating the implantation of a stimulation lead in the brain of a patient, wherein the stimulation lead comprises a plurality of electrodes, the apparatus comprising:
control circuitry configured to:
receive an indication that the lead is at a first position in the patient's brain,
determine a plurality of stimulation locations at which to apply stimulation, wherein at least one of the stimulation locations is not co-located with an electrode,
apply stimulation at each of the determined stimulation locations, wherein applying stimulation at the at least one location not co-located with an electrode comprises fractionalizing current to two or more electrodes to provide the stimulation at the determined stimulation location,
detect an evoked response evoked at a neural target for each of the stimulation locations, and
determine one or more of (i) whether to move the lead to a new position or (ii) to adjust stimulation parameters based on the evoked responses.

2. The apparatus of claim 1, wherein the detected evoked response occurs following an evoked compound action potential, ECAP.

3. The apparatus of claims 1 or 2, wherein at least two electrodes of the same polarity are used during at least one stimulation.

4. The apparatus of any of claims 1-3, wherein the evoked response is evoked resonant neural activity, ERNA.

5. The apparatus of claim 4, wherein the control circuitry is further configured to display an indication of the ERNAs for each of the stimulation locations on a user interface, UI.

6. The apparatus of claim 5, wherein the control circuitry is further configured to display an indication of the ERNA interpolated between the stimulation locations on the user interface.

7. The apparatus of claim 5, wherein the indications of the ERNAs for each of the stimulation locations comprises an indication of the ERNA amplitudes.

8. The apparatus of claim 7, wherein the UI comprises a representation of the stimulation lead and wherein the indications of the ERNA amplitudes are displayed upon the representation of the stimulation lead at positions corresponding to the corresponding stimulation locations.

9. The apparatus of any of claims 1-8, wherein the control circuitry is further configured to determine a prediction of efficacy for stimulation using the lead at the first position.

10. The apparatus of any of claims 1-8, wherein the control circuitry is further configured to determine a relative position of the first position with respect to the neural target.

11. The apparatus of claim 10, wherein determining the relative position of the first position with respect to the neural target comprises estimating a distance between the first position and the neural target.

12. The apparatus of claim 10, wherein determining the relative position of the first position with respect to the neural target comprises determining a direction from the first position to the neural target.

13. The apparatus of claim 10, wherein the control circuitry is further configured to determine an orientation of the lead with respect to the neural target.

14. The apparatus of any of claims 1-13, wherein using the electrodes to apply stimulation at the plurality of stimulation locations comprises applying monopolar stimulation.

15. The apparatus of claim 14, wherein applying monopolar stimulation comprises applying a first set of monopolar stimulation pulses of a first polarity and a second set of monopolar stimulation pulses of a second polarity.

## Patentansprüche

1. Vorrichtung zum Erleichtern der Implantation einer Stimulationsleitung in das Gehirn eines Patienten, wobei die Stimulationsleitung eine Mehrzahl von Elektroden aufweist, wobei die Vorrichtung aufweist:
eine Steuerschaltung, die dafür konfiguriert ist:
eine Anzeige zu empfangen, dass sich die Leitung an einer ersten Position im Gehirn des Patienten befindet;
eine Mehrzahl von Stimulationsorten zu bestimmen, an denen eine Stimulation bereitgestellt werden soll, wobei mindestens einer der Stimulationsorte nicht an der gleichen Stelle wie eine Elektrode angeordnet ist;
eine Stimulation an jedem der bestimmten Stimulationsorte bereitzustellen, wobei das Bereitstellen einer Stimulation an dem mindestens einen Ort, der sich nicht an der gleichen Stelle wie eine Elektrode befindet, das Aufteilen des Stroms auf zwei oder mehr Elektroden aufweist, um die Stimulation an dem bestimmten Stimulationsort bereitzustellen;
eine an einem neuronalen Ziel für jeden der Stimulationsorte evozierte Reaktion zu erfassen; und
eine oder mehrere der folgenden Optionen zu bestimmen: (i) ob die Leitung an eine neue Position bewegt werden soll, oder (ii) die Stimulationsparameter basierend auf den evozierten Reaktionen einzustellen.

2. Vorrichtung nach Anspruch 1, wobei die erfasste evozierte Reaktion nach einem evozierten Summen-Aktionspotential, ECAP, auftritt.

3. Vorrichtung nach Anspruch 1 oder 2, wobei während mindestens einer Stimulation mindestens zwei Elektroden der gleichen Polarität verwendet werden.

4. Vorrichtung nach einem der Ansprüche 1 bis 3, wobei die evozierte Reaktion eine evozierte resonante neuronale Aktivität, ERNA, ist.

5. Vorrichtung nach Anspruch 4, wobei die Steuerschaltung ferner dafür konfiguriert ist, eine Anzeige der ERNAs für jeden der Stimulationsorte auf einer Benutzeroberfläche, UI, anzuzeigen.

6. Vorrichtung nach Anspruch 5, wobei die Steuerschaltung ferner dafür konfiguriert ist, eine Anzeige der zwischen den Stimulationsorten interpolierten ERNAs auf der Benutzeroberfläche anzuzeigen.

7. Vorrichtung nach Anspruch 5, wobei die Anzeigen der ERNAs für jeden der Stimulationsorte eine Anzeige der ERNA-Amplituden aufweisen.

8. Vorrichtung nach Anspruch 7, wobei die Benutzeroberfläche eine Darstellung der Stimulationsleitung aufweist, und wobei die Anzeigen der ERNA-Amplituden auf der Darstellung der Stimulationsleitung an Positionen angezeigt werden, die den entsprechenden Stimulationsorten entsprechen.

9. Vorrichtung nach einem der Ansprüche 1 bis 8, wobei die Steuerschaltung ferner dafür konfiguriert ist, eine Vorhersage der Wirksamkeit der Stimulation unter Verwendung der Leitung an der ersten Position zu bestimmen.

10. Vorrichtung nach einem der Ansprüche 1 bis 8, wobei die Steuerschaltung ferner dafür konfiguriert ist, eine relative Position der ersten Position in Bezug auf das neuronale Ziel zu bestimmen.

11. Vorrichtung nach Anspruch 10, wobei das Bestimmen der relativen Position der ersten Position in Bezug auf das neuronale Ziel das Schätzen eines Abstands zwischen der ersten Position und dem neuronalen Ziel aufweist.

12. Vorrichtung nach Anspruch 10, wobei das Bestimmen der relativen Position der ersten Position in Bezug auf das neuronale Ziel das Bestimmen einer Richtung von der ersten Position zum neuronalen Ziel aufweist.

13. Vorrichtung nach Anspruch 10, wobei die Steuerschaltung ferner dafür konfiguriert ist, eine Ausrichtung der Leitung in Bezug auf das neuronale Ziel zu bestimmen.

14. Vorrichtung nach einem der Ansprüche 1 bis 13, wobei das Verwenden der Elektroden zum Bereitstellen einer Stimulation an der Mehrzahl von Stimulationsorten das Bereitstellen einer monopolaren Stimulation aufweist.

15. Vorrichtung nach Anspruch 14, wobei das Bereitstellen einer monopolaren Stimulation das Bereitstellen eines ersten Satzes von monopolaren Stimulationsimpulsen einer ersten Polarität und eines zweiten Satzes von monopolaren Stimulationsimpulsen einer zweiten Polarität aufweist.

## Revendications

1. Appareil destiné à faciliter l'implantation d'un fil de stimulation dans le cerveau d'un patient, dans lequel le fil de stimulation comprend une pluralité d'électrodes, l'appareil comprenant :
une circuiterie de commande configurée pour :
recevoir une indication disant que le fil se trouve en une première position dans le cerveau du patient,
déterminer une pluralité d'emplacements de stimulation auxquels appliquer une stimulation, dans lequel au moins l'un des emplacements de stimulation n'est pas colocalisé avec une électrode,
appliquer une stimulation à chacun des emplacements de stimulation déterminés, dans lequel l'application d'une stimulation à le au moins un emplacement non colocalisé avec une électrode comprend le fractionnement d'un courant vers deux ou plusieurs électrodes pour fournir la stimulation en l'emplacement de stimulation déterminé,
détecter une réponse évoquée qui est évoquée en une cible neuronale pour chacun des emplacements de stimulation, et
déterminer un ou plusieurs parmi i) déplacer ou non le fil vers une nouvelle position ou ii) ajuster des paramètres de stimulation sur la base des réponses évoquées.

2. Appareil selon la revendication 1, dans lequel la réponse évoquée détectée survient à la suite d'un potentiel d'action composé évoqué, ECAP (*evoked compound action potential*).

3. Appareil selon les revendications 1 ou 2, dans lequel au moins deux électrodes de la même polarité sont utilisées pendant au moins une stimulation.

4. Appareil selon l'une quelconque des revendications 1-3, dans lequel la réponse évoquée est une activité neuronale résonante évoquée, ERNA (*evoked resonant neural activity*).

5. Appareil selon la revendication 4, dans lequel la circuiterie de commande est en outre configurée pour afficher une indication des ERNA pour chacun des emplacements de stimulation sur une interface utilisateur, UI (*user interface*).

6. Appareil selon la revendication 5, dans lequel la circuiterie de commande est en outre configurée pour afficher une indication de l'ERNA interpolée entre les emplacements de stimulation sur l'interface utilisateur.

7. Appareil selon la revendication 5, dans lequel les indications des ERNA pour chacun des emplacements de stimulation comprend une indication des amplitudes ERNA.

8. Appareil selon la revendication 7, dans lequel l'UI comprend une représentation du fil de stimulation et dans lequel les indications des amplitudes ERNA sont affichées sur la représentation du fil de stimulation en des positions correspondant aux emplacements de stimulation correspondants.

9. Appareil selon l'une quelconque des revendications 1-8, dans lequel la circuiterie de commande est en outre configurée pour déterminer une prévision d'efficacité pour une stimulation à l'aide du fil dans la première position.

10. Appareil selon l'une quelconque des revendications 1-8, dans lequel la circuiterie de commande est en outre configurée pour déterminer une position relative de la première position par rapport à la cible neuronale.

11. Appareil selon la revendication 10, dans lequel la détermination de la position relative de la première position par rapport à la cible neuronale comprend l'estimation d'une distance entre la première position et la cible neuronale.

12. Appareil selon la revendication 10, dans lequel la détermination de la position relative de la première position par rapport à la cible neuronale comprend la détermination d'une direction depuis la première position vers la cible neuronale.

13. Appareil selon la revendication 10, dans lequel la circuiterie de commande est en outre configurée pour déterminer une orientation du fil par rapport à la cible neuronale.

14. Appareil selon l'une quelconque des revendications 1-13, dans lequel l'utilisation des électrodes pour appliquer une stimulation à la pluralité d'emplacements de stimulation comprend l'application d'une stimulation monopolaire.

15. Appareil selon la revendication 14, dans lequel l'application d'une stimulation monopolaire comprend l'application d'un premier ensemble d'impulsions de stimulation monopolaire d'une première polarité et d'un deuxième ensemble d'impulsions de stimulation monopolaire d'une deuxième polarité.
